# EUROPÄISCHE PATENTANMELDUNG

(11) **EP 3 443 996 A1**
(43) Veröffentlichungstag der Anmeldung: **20.02.2019**
(21) Anmeldenummer: 17186867.2
(22) Anmeldetag: 18.08.2017
(51) Int. Cl.: A61M 5/142

(54) **PATCH-PUMPE**

(71) Anmelder: TecPharma Licensing AG, 3401 Burgdorf (CH)
(72) Erfinder: BURI, Thomas, 3400 Burgdorf (CH); HOFER, Christophe, 3400 Burgdorf (CH); HOSTETTLER, Patrick, 3415 Hasle (CH); KOEHLI, Andreas, 2563 Ipsach (CH); KUEHNI, Florian, 3007 Bern (CH); LABUDDE, Marc, 3095 Spiegel (CH); LEUZINGER, Thomas, 3110 Münsingen (CH); STAUB, Seline, 8604 Volketswil (CH); STECK, Jürg, 3422 Kirchberg (CH); STREIT, Ursina, 3322 Schönbühl (CH)

(57) **Zusammenfassung**

Einwegmodul (Disposable) (20), welches mit einem Mehrwegmodul (Reusable) (10) gekoppelt werden kann, um im gekoppelten Zustand eine Pumpe zur dosierten Abgabe einer Substanz an einen Anwender zu bilden, mit einem Gehäuse, das folgende Komponenten umgibt:
- ein Reservoir (22) zur Aufnahme der abzugebenden Substanz; und
- eine ausfahrbare Kanüle (23), insbesondere eine Hartkanüle und/oder eine Softkanüle, die zumindest nach der Insertion oder dauerhaft, also auch vor einem Insertionsvorgang, mit dem Reservoir (22) in Fluidverbindung steht oder stehen.

## Beschreibung

Die Erfindung betrifft eine Patch-Pumpe zur dosierten Abgabe einer Substanz, wie z. B. Insulin, an einen Benutzer. Die Pumpe weist eine Einwegkomponente (auch als "Disposable" bezeichnet), welche z. B. Verbrauchselemente wie ein Reservoir für die abzugebende Substanz, eine Kanüle, eine Batterie und ein Klebepflaster oder eine Haftfläche umfasst, und eine wiederverwendbaren Komponente (auch als "Reusable" bezeichnet), welche beispielsweise einen Dosier- oder Abgabemechanismus, z. B. im Form einer Pumpe, eine Energieversorgung und elektronische Komponenten, wie z. B. eine Steuereinheit beinhaltet, auf

### HINTERGRUND DER ERFINDUNG

Bestimmte Erkrankungen können durch eine kontrollierte Abgabe einer Substanz an den Körper eines Patienten behandelt werden, wobei die Substanzabgabe gesteuert z. B. kontinuierlich oder zu vorgegebenen Zeiten oder Intervallen erfolgt. Beispielsweise kann Diabetes durch kontrollierte Abgabe von Insulin in den Körper eines Patienten behandelt werden. Eine Möglichkeit der kontrollierten Substanzabgabe ist die Abgabe durch eine externe Pumpe an einen Patienten, welche z. B. an dem Patienten getragen oder auf dessen Haut aufgeklebt wird, wobei die abzugebende Substanz aus der Pumpe durch einen in den Körper des Patienten eingebrachten Katheter abgegeben wird.

Aus der WO 2006/121921 A2 ist eine Abgabevorrichtung bestehend aus einem Gehäuseteil und einem separierbaren Wegwerfteil bekannt, wobei das Wegwerfteil am Patienten angebracht werden kann und ein Infusat enthält, während im Gehäuseteil die anderen erforderlichen Komponenten, wie z. B. die gesamte Steuerelektronik, Batterien, und ein Antrieb vorhanden sind.

WO 2009/125398 A2 offenbart eine Fluidabgabevorrichtung mit einem wiederverwendbaren Teil mit einem Controller und einem Antriebsmechanismus und einem Einweg-Teil mit einem Reservoir für ein Fluid, welche miteinander gekoppelt werden können.

Die US 8,905,972 B2 offenbart eine Infusionspumpe, welche aus einer Basisplatte, einer Kartusche und einem wiederverwendbaren Teil zusammengesetzt werden kann, wobei ein Inserter auf die zusammengesetzte Pumpe aufgesetzt wird, um eine Kanüle durch ein Durchgangsloch der Pumpe hindurch zu setzen.

### DEFINITIONEN

### Einwegmodul (Disposable)

Ein Einwegmodul (auch als Einwegkomponente oder Wegwerfmodul oder Disposable bezeichnet) umfasst im Ausgangszustand innerhalb des Disposable-Gehäuses vorzugsweise Verbrauchsartikel, wie beispielsweise ein befüllbares oder vorgefülltes Reservoir für die abzugebende Substanz (z. B. Insulin), beispielsweise mit einem Füllvolumen von bis zu 2 ml, eine bewegbare oder elastische Kanüle, welche in den Patienten zur Abgabe der Substanz eingebracht werden kann, und an einer Außenfläche des Gehäuses einen Haftbereich oder Klebepflaster, um die Einwegkomponente z. B. auf der Haut eines Patienten ankleben zu können. Optional umfasst das Einwegmodul weitere Komponenten, wie z. B. einen Einstech- oder Insertionsmechanismus für die Kanüle, welche z. B. eine Softkanüle sein kann oder eine Energiequelle z. B. eine Einwegbatterie oder Knopfzelle.

Der Insertions- oder Injektionsmechanismus für die Softkanüle kann innerhalb des Disposable-Gehäuses als automatischer Insertionsmechanismus ausgebildet sein, welcher z. B. auch die für die Insertion erforderliche Energie speichert, z. B. in Form einer gespannten oder komprimierten Feder. Der Auslösemechanismus für die Insertion, der z. B. die Freigabe der gespeicherten Insertionsenergie steuern oder veranlassen kann, kann z. B. ebenfalls im Disposable vorgesehen sein und z. B. vom Reusable angesteuert werden.

Optional kann eine Druckausgleichsmembran im Disposable vorgesehen sein, um z. B. Druckschwankungen auszugleichen, die zu einem ungewollten Ausschütten führen würden.

Eine Batterie kann wie erwähnt im Disposable vorgesehen sein, wie z. B. eine oder mehrere nicht-aufladbare Einwegbatterien, z. B. eine oder mehrere Knopfzellen, welche zum elektrischen Aufladen eines aufladbaren Speicherelements für elektrische Energie oder Akkus im Reusable dient. Die Batterie(n) kann fest im Disposable eingebaut und z. B. unlösbar mit diesem verbunden sein, so dass diese nicht vom Disposable getrennt oder ausgewechselt werden kann.

Das Disposable oder dessen Komponenten sind z. B. in einem Disposable-Gehäuse untergebracht, das eine Verbindungsschnittstelle zum Mehrwegmodul oder Reusable aufweist. Dabei kann die Unterseite des Gehäuses den Haftbereich oder das z. B. mit einer abziehbaren Schutzfolie abgedeckten Klebepflaster aufweisen.

### Mehrwegmodul (Reusable)

Das Mehrwegmodul (auch als wiederverwendbares Modul oder Reusable bezeichnet) umfasst vorzugsweise Komponenten, welche nicht verbraucht und für mehrere Abgabevorgänge verwendet werden können. Vorzugsweise ist das Mehrwegmodul oder Reusable mit denjenigen Komponenten bestückt, welche im Zusammenhang mit einem Disposable, welches mit dem Reusable gekoppelt oder verbunden werden kann, eine dosierte Abgabe einer Substanz ermöglichen. Dabei können die im Disposable enthaltenen verbrauchbaren Elemente, wie z. B. die abzugebende Substanz und elektrische Energie, vom Reusable aufgenommen und/oder mittels des Reusables aufgenommen oder abgegeben werden, was bei einem Reusable auch für mehrere sequentiell mit dem Reusable gekoppelte Disposables möglich ist. Beispielsweise kann das Reusable vom Disposable elektrische Energie aufnehmen, und so auf das Disposable einwirken, dass die im Disposable enthaltene Substanz unter Steuerung durch das Reusable kontrolliert aus dem Disposable z. B. durch eine Kanüle an den Benutzer abgegeben wird. Das Reusable soll vorzugsweise so ausgestaltet sein, dass es mit mehreren Disposables nacheinander gekoppelt werden kann und jeweils die für den Betrieb oder die dosierte Abgabe erforderliche Energie vom Disposable übertragen bekommt, wobei die im Disposable enthaltenen Substanz entweder direkt aus dem Disposable durch einen im Reusable vorhandenen Antrieb oder Motor aus dem Disposable z. B. durch eine Kanüle abgegeben wird oder alternativ zum Reusable übertragen und aus diesem abgegeben wird.

Das Reusable enthält vorzugsweise einen Antrieb, welcher z. B. als Motor oder Elektromotor ausgebildet sein kann. Dabei kann der Motor unmittelbar oder mittelbar z. B. über ein Getriebe und/oder eine Kolbenstange und/oder eine Dosiermechanik, wie z. B. ein Vorschubgewinde, so auf das Reservoir der abzugebenden Substanz, z. B. auf einen verschiebbaren Stopfen einer Ampulle, einwirken, dass die Substanz gesteuert oder dosiert abgegeben werden kann, wenn z. B. das Reusable mit dem Disposable gekoppelt ist. Beispielsweise kann durch eine Ansteuerung des Motors eine definierte Substanzmenge aus dem Reservoir z. B. kontinuierlich mit einer durch das Reusable bestimmten Rate und/oder zu durch das Reusable vorgegebenen Zeiten abgegeben werden.

Das Reusable weist vorzugsweise elektronische Komponenten zur Steuerung des Antriebs oder Motors auf. Weiterhin kann optional auch ein Speicherelement und/oder eine Datenübertragungseinrichtung oder Funkverbindungselement im Reusable vorgesehen sein, welches z. B. mit einem externen Steuerelement, wie z. B. einem Mobiltelefon, einem Computer oder einer auf einem Gerät laufenden Pumpensteuerungs-App Daten austauschen kann.

Ebenso kann das Reusable z. B. die Insertionsvorrichtung des Disposables ansteuern oder auslösen, z. B. einen auf die Kanüle wirkenden Energiespeicher oder eine Kraft oder Feder freigeben, um eine automatische Kanülen-Insertion durchzuführen.

Das Reusable weist eine Energieversorgung auf, wie z. B. ein aufladbares Element oder Akkumulator, welcher z. B. von einer mit dem Reusable gekoppelten im Disposable enthaltenen Batterie elektrisch aufgeladen werden kann. Die Energieversorgung kann z. B. den erwähnten Elektromotor und die optional vorhandenen elektronischen Komponenten mit Strom versorgen. Das aufladbare Element oder Akkumulator kann fest und z. B. nicht austauschbar oder auch auswechselbar im Reusable eingebaut sein.

Optional kann eine Druckausgleichmembran vorgesehen sein.

Das Reusable oder dessen Komponenten sind z. B. in einem Reusable-Gehäuse untergebracht, das eine Verbindungsschnittstelle zum Einwegmodul oder Disposable aufweist.

### Verbindungsschnittstelle

Das Reusable kann mit dem Disposable mechanisch und/oder elektrisch über eine Schnittstelle verbunden oder gekoppelt werden. Ebenso kann z. B. ein Datenaustausch zwischen Reusable und Disposable möglich sein, z. B. über eine Funkverbindung oder mittels der elektrischen Schnittstelle.

Die **mechanische Schnittstelle oder Verbindung oder Kopplung** zwischen Disposable und Reusable kann eine lösbare mechanische Verbindung z. B. in Gestalt eines Bajonettverschlusses sein. Dabei kann die mechanische Verbindungsschnittstelle z. B. so ausgebildet sein, dass ein Bediener die beiden Baugruppen Disposable und Reusable in korrekter Ausrichtung im Bereich der Verbindung, wie z. B. der Bajonettverbindung, aneinander legen oder ineinander fügt oder fügen muss und anschließend um einen vorgegebenen Winkel relativ zueinander verdreht oder verdrehen muss, bis die Module miteinander verrastet sind. Reusable und Disposable können z. B. nach einer relativ zueinander ausgeführten Drehbewegung automatisch miteinander oder ineinander verrasten oder verschnappen und optional auch nach der Verrastung oder Verschnappung einen elektrischen Kontakt herstellen. Ein Bajonettverschluss oder Gewindeverschluss kann durch den Einsatz einer gewindeförmigen Geometrie das Zusammenziehen von Reusable und Disposable beim Verbinden oder Schließen dieser Komponenten ermöglichen und so eine spielfreie Verbindung realisieren, welche durch einen Benutzer mit geringem Kraftaufwand hergestellt werden kann.

Vorzugsweise besteht eine elektrische Verbindung zwischen Disposable und Reusable nur, wenn diese miteinander verriegelt sind. Beispielsweise können die elektrischen Kontakte am Reusable und Disposable so dimensioniert werden, dass diese noch keine elektrische Verbindung bereitstellen, also z. B. noch einen Abstand voneinander haben, wenn Disposable und Reusable noch nicht so weit zusammengezogen oder z. B. verschraubt sind, dass eine sichere Verbindung oder Verrastung oder Verriegelung hergestellt worden ist.

Um die mechanische Verbindung oder Verriegelung wieder lösen zu können, kann ein Feststell- oder Freigabeelement, wie beispielsweise ein Schiebeelement vorgesehen sein, welches betätigt werden kann, um die Verrastung oder Verriegelung der Baugruppen zu sichern oder wieder freizugeben, so dass beispielsweise die Baugruppen entgegen der Schließrichtung relativ zueinander verdreht werden können, um die mechanische Kopplung oder Verbindung zu lösen, so dass Disposable und Reusable voneinander getrennt werden können. Das Feststell- oder Freigabeelement, wie z. B. ein Schiebeelement, kann elektronisch verriegelt sein, so dass die Freigabe der mechanischen Verriegelung elektronisch z. B. durch eine ControlApp gesteuert werden kann.

Das Feststellelement zum Sichern oder Arretieren der Verbindung zwischen Reusable und Disposable kann beispielsweise am Reusable oder alternativ am Disposable vorgesehen sein. Dabei kann das Feststellelement, wie z. B. ein Schiebeschalter, durch eine Feder in einer vorgegebenen Position gehaltet werden, so dass das Feststellelement beispielsweise in der Ausgangsposition ein Verrasten zwischen Reusable und Disposable ermöglicht und ein ungewolltes Öffnen dieser Verbindung durch die Vorspannung der Feder verhindert. Ein Benutzer muss das Feststellelement oder einen Schiebeschalter bewusst gegen die Feststellkraft oder Federkraft verschieben und im Falle einer gleichzeitig vorgesehenen Gewindeverbindung oder Bajonettverbindung auch gleichzeitig eine Drehbewegung ausführen, um die Verbindung zwischen Disposable und Reusable öffnen zu können.

Die mechanische Schnittstelle zwischen Reusable und Disposable kann spielfrei ausgestaltet sein. Dies ist vorteilhaft, da die mechanische Verbindung zwischen Reusable und Disposable z. B. im Betrieb der Pumpe die Ausschüttkräfte der Spritzenpumpe oder des Motors aufnimmt. Durch eine spielfreie mechanische Verbindung kann sichergestellt werden, dass die Menge der abzugebenden Substanz genau gesteuert werden kann und nicht fälschlicherweise durch eine von außen aufgebrachte Kraft beeinflusst wird.

Vorzugsweise ist die Schnittstelle zumindest im Bereich der Dosiermechanik oder Ausschüttmechanik wasserdicht.

Das Reusable ist vorzugsweise insgesamt oder an der mechanischen oder Kopplungs-Schnittstelle zum Disposable aus einem verschleißarmen und/oder vergleichsweise hartem Material, wie z. B. harter Kunststoff und/oder Metall, gefertigt. Das Disposable ist vorzugsweise an der mechanischen Schnittstelle zum Reusable aus einem vergleichsweise weichem Material gefertigt, welches durch plastische Verformung einen Toleranzausgleich bieten kann und eine spielfreie Verbindung mit dem Reusable ermöglicht. Der restliche Teil des Disposables kann auch aus einem harten Material gefertigt sein.

Die Verbindung kann eine Dichtung z. B. aus einer weichen Komponente, wie beispielsweise Silikon, aufweisen, welche z. B. direkt an einem Gehäuseteil befestigt ist. Wird die Dichtung am Disposable angebracht, so kann eine hohe Funktionssicherheit ermöglicht werden, da diese Dichtung bei jedem Wechsel des Disposables durch eine neue ersetzt wird.

Eine **elektronische Schnittstelle** zwischen Reusable und Disposable kann eine oder mehrere der folgenden Funktionen realisieren:
1. Aufwecken der Reusable-Elektronik aus dem Lagerzustand beim Anschließen des Disposable
2. Übertragung der elektrischen Energie zum Aufladen des Akkus vom Disposable, welcher beispielsweise eine Einwegbatterie enthält, zum Reusable
3. Überprüfung des Vorhandenseins eines Disposables am Reusable
4. Lesen und Schreiben von Disposable-Informationen, wie z. B. Auslesen und/oder Prüfen des Ablaufdatums des angeschlossenen Disposables, Typenprüfung (z. B. Version, Reservoirgröße) des Disposables durch das Reusable oder eine Software des Reusables, wobei optional davon abhängig eine Anpassung der Ausschüttung vorgenommen werden kann
5. Signal zum Auslösen oder Freigeben des Insertions- oder Einstichmechanismus der Kanüle vom Reusable zum Disposable
6. Übertragung der elektrischen Energie für die Auslösung des Insertions- oder Einstichmechanismus vom Reusable zum Disposable
7. Überprüfung ob das Einstechen der Kanüle oder Nadel im Disposable ausgeführt wurde, durch das Reusable.

Die elektronische Schnittstelle kann beispielsweise durch elektrische Kontakte gebildet werden, was vorzugsweise der Fall ist, wenn eine elektrische Energie vom Disposable auf den Reusable, z. B. zum Aufladen eines Reusable-Akkus, übertragen wird. Steuerbefehle können z. B. ebenfalls über elektrische Kontakte oder auch drahtlos z. B. durch Funk übertragen werden.

### Kanüle

Eine Kanüle ist eine Fluidverbindung, wie z. B. eine starre, nicht-elastische oder elastische Röhre, Nadel oder ein Schlauch, durch welche eine Substanz oder ein Fluid transportiert werden kann. Die Kanüle steht vorzugsweise zumindest nach der Insertion, d.h. nach dem Ausstoßen der Kanüle aus der Pumpe, also z. B. aus dem Disposable-Gehäuse der Pumpe, oder dem Eindringen der Kanülenspitze in einen Patienten, in Fluidverbindung mit einem Reservoir, welches die abzugebende Substanz enthält. Dabei kann gemäß einer Ausführungsform der vorliegenden Erfindung die Kanüle auch dauerhaft oder kontinuierlich, also z. B. schon vor Durchführung des Insertionsvorganges, mit dem Reservoir in Verbindung stehen. Es wird also z. B. nicht erst während der Insertion eine Fluidverbindung vom Reservoir zur Kanüle hergestellt.

Die Kanüle kann z. B. eine Hartkanüle, z. B. aus einem Metall bestehend, sein, durch welche eine Substanz hindurch befördert werden kann. Die Hartkanüle weist vorzugsweise ein vorderes zur Insertion geeignetes spitzes Ende mit einer Durchtrittsöffnung auf, welche z. B. nach Art einer bekannten Spritze durch eine Haut hindurchgestochen werden kann und durch welche eine Substanz abgegeben werden kann.

Gemäß einer Ausführungsform kann die Kanüle als Softkanüle ausgebildet sein, also aus einem weichen und verformbaren oder biegbaren elastischen Material gefertigt sein, welches z. B. auf an sich bekannte Art dadurch eingestochen werden kann, dass innerhalb der Softkanüle eine Insertionsnadel oder auch Hartkanüle vorgesehen ist, die aus der vorderen Öffnung der Softkanüle herausragt und welche die eigentliche Insertion durchführt, also z. B. durch die Haut eines Patienten sticht, und während des Insertions- oder Einstichvorganges die um die Hartkanüle herumliegende Softkanüle mit sich führt, sodass diese ebenfalls eingestochen oder eingebracht wird. Nach erfolgter Insertion kann die Softkanüle auf bekannte Art an der Insertionsstelle verbleiben, z. B. indem diese gegen ein Zurückziehen gesichert oder z. B. am Gehäuse verrastet wird, wobei die Nadel oder Hartkanüle aus der Softkanüle zurückgezogen wird, sodass die Nadel oder Hartkanüle nicht mehr eingestochen ist.

Gemäß einer Ausführungsform kann die Hartkanüle auch nach der Insertion in der Softkanüle in einem zurückgezogenen Zustand verbleiben, sodass z. B. eine in einem Reservoir enthaltene Substanz durch die z. B. fest mit dem Reservoir verbundene oder fluidisch gekoppelte Hartkanüle hindurch fließt, welche die Substanz an ihrer vorderen Austrittsöffnung an die sie umgebende Softkanüle abgibt, in welcher die Hartkanüle nach der Insertion verblieben ist, sodass die Substanz durch die Softkanüle hindurch an die Insertionsstelle und z. B. durch die Hautoberfläche eines Patienten hindurch transportiert werden kann, um an der Austrittsöffnung der Weichkanüle, welche nach der Insertion z. B. in einem Patienten ist, abgegeben zu werden.

Nach der Insertion bleiben gemäß einem Ausführungsbeispiel sowohl Hartkanüle als auch Softkanüle in Fluidverbindung mit dem Reservoir, wobei die Hartkanüle nach erfolgter Insertion relativ zur Softkanüle verschoben, also z. B. in dieser zurückgezogen oder in diese eingezogen wird.

Alternativ ist es möglich, dass eine Fluidverbindung zwischen Softkanüle und optional auch Hartkanüle zum Reservoir erst mit oder nach erfolgter Insertion hergestellt wird.

Nach der Insertion, also dem Einbringen der Kanüle (Hartkanüle und/oder Softkanüle), kann mit der Injektion, also der dosierten Abgabe einer Substanz durch die Kanüle, begonnen werden.

### Aspekte der Erfindung

Gemäß einem Aspekt betrifft die Erfindung ein Einwegmodul oder Disposable, welches mit einem Mehrwegmodul gekoppelt werden kann, so dass sich im zusammengesetzten Zustand eine funktionsfähige Pumpe zur dosierten Abgabe einer Substanz ergibt. Das Einwegmodul oder Disposable weist keinen Antrieb oder Motor und z. B. auch keine oder keine vollständige Steuerelektronik auf, der bzw. die z. B. ausschließlich im Reusable vorgesehen ist und enthält, vorzugsweise in einem Disposable-Gehäuse, ein vorgefülltes oder befüllbares Reservoir zur Aufnahme oder Speicherung der dosiert abzugebenden z. B. medizinisch wirksamen Substanz. In dem Gehäuse ist weiterhin eine Kanüle, insbesondere eine wie oben beschriebene Softkanüle und/oder Hartkanüle, vorgesehen, die zumindest nach der Insertion oder dauerhaft mit dem Reservoir in Fluidverbindung steht. Die Kanüle wird folglich nicht von extern dem Disposable hinzugefügt, sondern ist bereits im Disposable vorhanden und kann z. B. mittels eines geeigneten ebenfalls in dem Gehäuse vollständig enthaltenen Insertionsmechanismus aus dem Disposable ausgeschoben oder ausgestoßen werden, um so eine Insertion durchzuführen.

Da die Kanüle erfindungsgemäß bereits zusammen mit dem Reservoir im Disposable-Gehäuse vorhanden ist, kann die Kanüle z. B. mit großer Sicherheit steril gehalten werden, da ein Benutzer die Kanüle vor und zur Insertion nicht berühren kann.

Optional kann in dem Gehäuse auch der vollständige Insertionsmechanismus vorgesehen sein, mit welchem die Kanüle aus dem Gehäuse ausgeschoben werden kann. Alternativ könnte der Insertionsmechanismus auch zum Teil oder vollständig außerhalb des Gehäuses liegen und z. B. von extern, also von außerhalb des Gehäuses, eine Kraft zur Verfügung stellen, welche auf die Kanüle im Disposable-Gehäuse übertragen werden kann, um die Insertion durchzuführen. Gemäß einer Ausführungsform ist der Insertionsmechanismus zusammen mit dem Reservoir und der Kanüle, z. B. bestehend aus Softkanüle und Hartkanüle wie oben beschrieben, im Disposable-Gehäuse vorgesehen. Der Insertionsmechanismus kann z. B. ein Kraft- oder Energiespeicherelement, wie beispielsweise eine gespannte Feder oder Druckfeder, sein oder beinhalten, welche beispielsweise von einem Halteelement gehalten wird, sodass nach Lösen des Halteelements die in dem Kraft- oder Energiespeicher enthaltene Kraft oder Energie auf die Kanüle übertragen wird, um diese aus dem Disposable-Gehäuse auszustoßen. Beispielsweise kann eine von einem mechanischen Element gehaltene Druckfeder nach Lösung der Halteverbindung und Freigabe der Druckfeder auf die Kanüle, also z. B. auf die Hartkanüle und/oder Softkanüle, so einwirken, dass diese bedingt durch die Federkraft aus dem Disposable-Gehäuse ausgestoßen wird oder werden. Die Kraftübertragung von einem Insertionsmechanismus auf die Kanüle kann z. B. mechanisch, wie in der US 8,905,972 B2 beschrieben, ausgestaltet sein, wobei jedoch z. B. ein Federelement z. B. waagrecht innerhalb des Disposable-Gehäuses angeordnet sein kann.

Optional kann innerhalb des Gehäuses auch noch eine geladene Batterie oder Stromquelle, z. B. eine Einweg-Batterie oder Knopfzelle, vorgesehen sein, welche zum Aufladen eines aufladbaren elektrischen Elements oder Akkus des Reusables verwendet werden kann, wenn das Disposable elektrisch mit dem Reusable verbunden oder gekoppelt ist.

Durch das Integrieren einer Batterie in ein Einwegmodul, welches mit einem Mehrwegmodul gekoppelt werden kann und nach einer gewissen Zeit ausgewechselt wird, ist es nicht mehr erforderlich, dass eine Batterie am Reusable gewechselt werden muss. Das Reusable kann somit ungeöffnet in einem vorgefertigten z. B. verschlossenen und abgedichteten Zustand verbleiben und bekommt vom Disposable vorzugsweise die zur Abgabe der im Disposable vorhandenen Substanz erforderliche elektrische Energiemenge durch das Disposable mitgeliefert. Das Disposable kann mit vollständig oder teilweise entleerter Batterie und je nach Anwendungsfall teilweise oder vollständig geleertem Substanz-Reservoir nach einer Benutzungszeit ausgewechselt werden.

Die Disposable-Batterie kann eine Einwegbatterie, also z. B. eine nicht aufladbare Batterie, wie z. B. eine, zwei oder mehr Knopfzellen sein, welche z. B. fest im Disposable eingebaut, also z. B. fest mit einem Disposable-Gehäuse verbunden ist.

Das Disposable kann einen Insertionsmechanismus für eine im Disposable vorhandene Kanüle, wie z. B. eine Softkanüle, aufweisen. Dabei kann die zur Insertion erforderliche Insertionsenergie beispielsweise ebenfalls im Disposable gespeichert sein, z. B. in Gestalt einer oder mehrerer gespannten Federn, welche durch ein Sicherungselement so gesichert ist oder sind, dass die Insertion noch nicht ausgelöst wird. Das Sicherungselement kann z. B. durch einen Benutzer gesteuert durch eine Signaleingabe an einem Steuergerät, welches im Reusable ist oder mit dem Reusable in Verbindung steht, freigegeben werden, wobei das Reusable das Freigabesignal an das Disposable überträgt, um den automatischen Insertionsmechanismus auszulösen. Dabei sollte das Disposable mit dem Reusable gekoppelt und an geeigneter Stelle auf dem Patienten aufgebracht oder angeklebt sein. Alternativ kann das Insertions-Freigabesignal auch direkt am Reusable oder auch direkt am Disposable eingegeben werden.

Das Disposable weist vorzugsweise eine Klebefläche auf, welche zur Befestigung des Disposables auf einer Oberfläche, z. B. der Haut eines Benutzers, dient. Die Klebefläche kann mit einer Schutzfolie abgedeckt sein, so dass diese wie bei einem bekannten Pflaster erst durch Abnahme der Schutzfolie vorzugsweise unmittelbar vor dem Aufkleben freigelegt wird. Die Basisplatte des Disposables kann so ausgebildet sein, dass das Pflaster nur am Disposable befestigt oder angebracht ist.

Gemäß einem weiteren Aspekt betrifft die Erfindung ein Mehrwegmodul oder Reusable, welches einen Antrieb oder Motor aufweist, der elektrische Energie in mechanische Energie umsetzen kann, um z. B. auf ein Reservoir der abzugebenden Substanz eines mit dem Reusable gekoppelten Disposables einzuwirken, so dass die Substanz gesteuert und dosiert abgegeben werden kann. Das Reusable weist weiterhin einen aufladbaren elektrischen Speicher, z. B. eine Akku, auf, welcher z. B. von einer im Disposable enthaltenen Batterie aufgeladen werden kann, wenn das Disposable mit dem Reusable gekoppelt ist oder gekoppelt wird.

Vorzugsweise treibt der Motor oder Antrieb des Reusables einen Kolben oder eine Gewindestange an, welche auf das Reservoir des Disposables einwirken kann, um kontrolliert eine Substanz aus dem Reservoir abzugeben. Beispielsweise kann der Kolben oder die Gewindestange auf einen verschiebbaren Stopfen des Reservoirs einwirken und diesen z. B. in das Reservoir, wie z. B. eine Ampulle, einschieben, um eine darin enthaltene Substanz zu verdrängen und durch eine Reservoir-Abgabeöffnung abzugeben, welche z. B. eine Kanüle sein kann oder mit dieser verbunden ist, um die Substanz abzugeben.

Das aufladbare elektrische Energiespeicherelement oder Akku kann z. B. fest mit dem Reusable, z. B. dem Reusable-Gehäuse, oder auswechselbar mit dem Reusable verbunden sein.

In dem Reusable können weitere elektrische oder elektronische Komponenten vorhanden sein, welche vorzugsweise von dem Energiespeicher oder Akku mit Strom versorgt werden können und beispielsweise zur Steuerung des Motors dienen oder eine direkte oder Funk-Kommunikation mit einem externen Bedienmodul eines Benutzers, wie z. B. einem Computer oder Mobiltelefon, ermöglichen, so dass der Benutzer Informationen über das Reusable und/oder Disposable erhalten und angezeigt bekommen kann und Steuerbefehle an das Reusable und von diesem an das Disposable übertragen kann. Die im Reusable enthaltenen Steuerung kann z. B. den im Reusable enthaltenen Motor ansteuern und kann z. B. auch die Kanüleninsertion eines automatischen Insertionsmechanismus im Disposable auslösen oder freigeben, wenn das Disposable mit dem Reusable gekoppelt ist.

Gemäß einem weiteren Aspekt betrifft die Erfindung eine Pumpe oder Abgabevorrichtung zur Abgabe einer Substanz mit einem wie oben beschriebenen Reusable, welches mit einem wie oben beschriebenen Disposable gekoppelt oder verbunden ist.

Gemäß einem weiteren Aspekt betrifft die Erfindung eine Schnittstelle zwischen einem wie oben beschriebenen Reusable und einem wie oben beschriebenen Disposable, welche so ausgestaltet ist, das eine elektrische Energie oder ein Ladestrom vom Disposable zum Aufladen eines elektrischen Energiespeichers oder Akkus im Reusable übertragen werden kann. Hierzu ist vorzugsweise ein elektrischer Kontakt bestehend aus mindestens zwei separaten Leitungen vorgesehen.

Die Schnittstelle kann auch so ausgelegt sein, dass auch Daten zwischen Reusable und Disposable übertragen oder ausgetauscht werden können.

Vorteilhaft sind die elektrischen Kontakte so abgedichtet, dass keine Flüssigkeit an die Schnittstelle und vorzugsweise auch nicht an die elektronischen Komponenten des Disposables oder Reusables durch die Schnittstelle gelangen kann.

Gemäß einem weiteren Aspekt betrifft die Erfindung ein Verfahren zum Bereitstellen oder Betreiben einer Abgabevorrichtung oder Pumpe für eine Substanz bestehend aus einem wie oben beschriebenen Reusable und wie oben beschriebenen Disposable, wobei das Disposable mit dem Reusable gekoppelt werden kann oder gekoppelt wird und in gekoppeltem Zustand elektrische Energie vom Disposable auf das Reusable übertragen wird.

### Kurze Beschreibung der Zeichnungen

Nachfolgend wird die Erfindung unter Bezugnahme auf die beiliegenden Figuren beschrieben, welche Ausführungsformen der Erfindung zeigen.
- Figur 1: zeigt eine schematische Darstellung der Komponenten einer aus Disposable und Reusable zusammengesetzten Pumpe gemäß einer Ausführungsform;
- Figur 2: zeigt das Zusammenfügen von Disposable und Reusable gemäß einer Ausführungsform;
- Figur 3: zeigt eine Ausführungsform einer aus Disposable und Reusable zusammengesetzten Patch-Pumpe;
- Figur 4: zeigt eine schematische Darstellung einer Ausführungsform eines Disposables mit Schnittstelle zur elektrischen Kontaktierung;
- Figur 5: zeigt das Energieversorgungskonzept gemäß einer Ausführungsform der Erfindung.

### Beschreibung von Ausführungsbeispielen

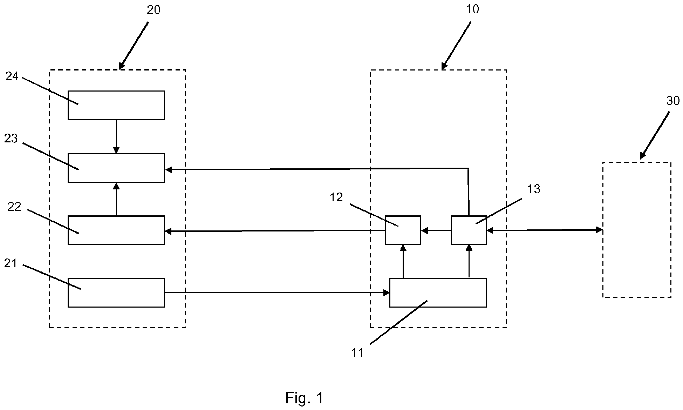

Figur 1 zeigt in schematischer Darstellung ein Ausführungsbeispiel eines Einwegmoduls oder Disposables 20, welches ein Reservoir 22 für eine abzugebende Substanz aufweist, welches vorgefüllt oder mittels einer Befüllöffnung befüllbar ausgestaltet sein kann. Das Reservoir 22 steht dauerhaft, also unterbrechungsfrei, schon im Ausgangszustand vor einer Insertion mit einer Kanüle 23 in Fluidverbindung, sodass aus dem Reservoir 22 eine Substanz durch die Kanüle 23 abgegeben werden kann.

Die Kanüle 23 kann mittels eines im Gehäuse des Disposables 20 vorgesehenen Kanülen-Insertionsmechanismus 24 z. B. aus einem Disposable-Gehäuse heraus ausgeschoben oder ausgestoßen werden, um so eine Insertion durchzuführen und die Kanüle 23 z. B. durch die Haut eines Benutzers einzustechen.

Im Disposable 20, also beispielsweise vom Disposable-Gehäuse umschlossen und/oder mit diesem fest verbunden, z. B. darin eingegossen, ist eine Batterie 21 z. B. in Gestalt einer Einwegbatterie oder Knopfzelle vorgesehen.

Ein Mehrwegmodul oder Reusable 10 weist in der gezeigten Ausführungsform einen Speicher für elektrische Energie in Gestalt eines wieder aufladbaren Akkus 11 auf, welcher, wenn Disposable 20 und Reusable 10 elektrisch gekoppelt sind, von der Batterie 21 des Disposables 20 aufgeladen werden kann. Der Akku 11 dient zur Stromversorgung des im Reusable 10 enthaltenen Motors 12 sowie der Steuerung 13, welche zur Ansteuerung des Motors 12 dient und Daten mit einem externen Steuergerät 30 z. B. über eine Funkverbindung austauschen kann. Auf dem externen Steuergerät 30 kann z. B. eine Software (Control App) ausgeführt werden, welche einem Benutzer die berührungslose Interaktion mit dem Reusable 10 und dem damit gekoppelten Disposable 20 ermöglicht.

Der Motor 12 kann mechanisch auf das Reservoir 22 einwirken, um eine darin enthaltene Substanz zu verdrängen, wenn z. B. über die auf dem externen Steuergerät 30 laufende ControlApp entsprechende Signale an die Steuerung 13 übertragen wurden und die Steuerung 13 den Motor 12 entsprechend ansteuert.

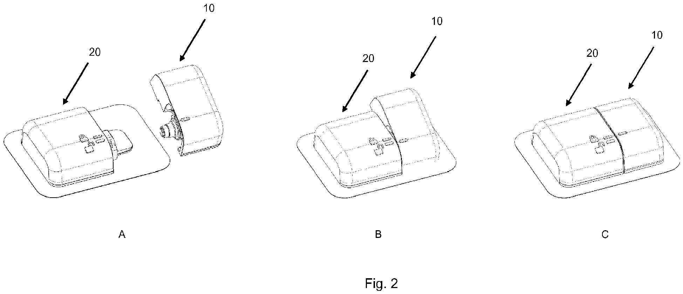

Die Figuren 2A bis 2C zeigen das Verbinden des Disposable 20 mit dem Reusable 10 am Ausführungsbeispiel eines Bajonettverschlusses. Dabei werden die zunächst wie in Figur 2A gezeigt getrennt vorliegenden Komponenten Reusable 10 und Disposable 20 zusammengesteckt, wie in Figur 2B gezeigt, und anschließend zur festen und spielfreien Verbindung relativ zueinander um den Bajonettverschluss gedreht, bis diese bündig aneinander anliegen, wie in Figur 2C gezeigt.

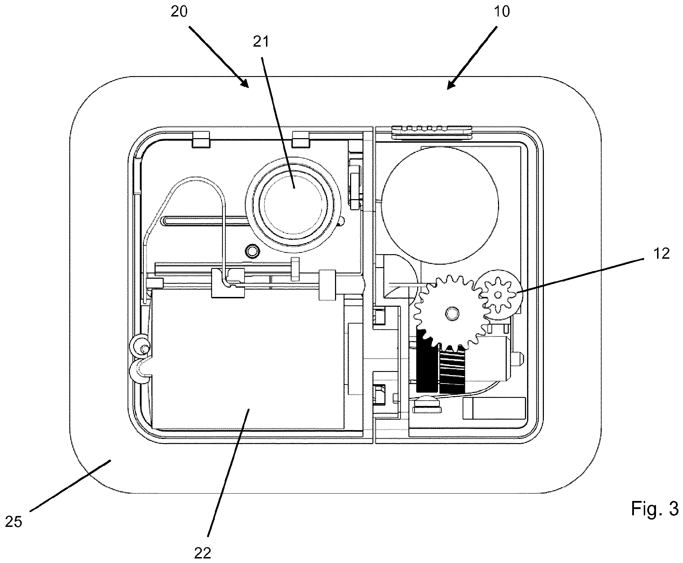

Figur 3 zeigt in Draufsicht ein Ausführungsbeispiel einer aus Disposable 20 und Reusable 10 zusammengesetzten Patch-Pumpe mit einem an der Unterseite des Disposable 20 angebrachten Pflasterelements 25 mit einer Haftfläche zum Anheften der zusammengesetzten Pumpe.

Gemäß einer Ausführungsform kann das Disposable 20 vor oder nach dem Zusammenfügen mit dem Reusable 10 befüllt werden. Hierzu kann z. B. eine an sich bekannte Spritze als Umfüllhilfe verwendet werden. Beispielsweise wird die abzugebende Substanz, wie beispielsweise ein Medikament, aus einem Primärpackmittel (vial) in die Spritze aufgezogen. Aus der aufgezogenen Spritze wird die Substanz oder das Medikament durch eine Einfüllöffnung in das Reservoir 22 des Disposables 20 abgegeben.

Nach dem Zusammensetzen von Reusable 10 und Disposable 20 oder nach der Bestätigung des Füllvorgangs kann z. B. die Füllmenge des Reservoirs 22 ermittelt werden. Hierzu kann beispielsweise ermittelt werden, wo sich der durch den Befüllvorgang des Reservoirs 22 verschobene Stopfen des Reservoirs 22 befindet, indem beispielsweise der Motor 12 so angesteuert wird, dass eine Kolbenstange in Richtung des Stopfens ausgefahren wird und festgestellt wird wann die Kolbenstange auf den Stopfen trifft, indem z. B. der Motorstrom gemessen wird.

Der Benutzer kann die Patch-Pumpe auf die Haut aufbringen und die Insertion der Nadel und nachfolgend auch die Insulinausschüttung auslösen.

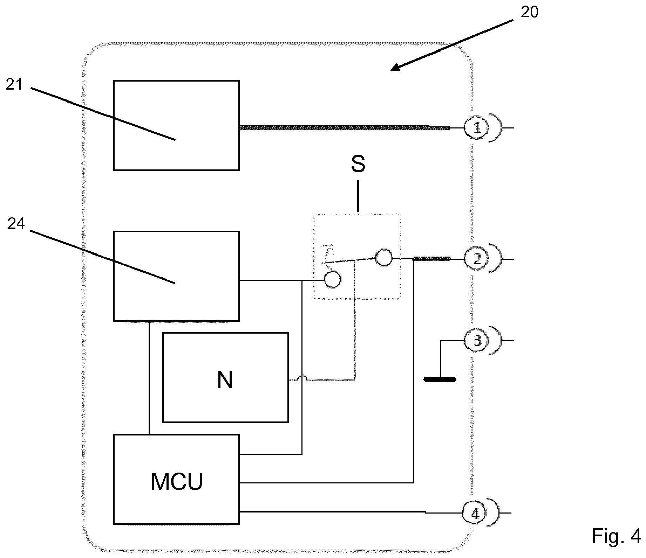

Figur 4 zeigt schematisch ein Ausführungsbeispiel eines Disposables 20 und dessen elektrische Kontakte 1 bis 4 an der Schnittstelle zum Reusable 10.

Die elektrischen Kontakte sind z. B. im Gehäuse des Disposables 20 eingespritzt. Die Kontaktierungsstelle der Schnittstelle ist im zusammengesteckten Zustand bevorzugt abgedichtet und bietet somit Schutz gegen Verschmutzung und Wasser. Am Reusable 10 können z. B. robuste Kontaktflächen vorgesehen sein. Am Disposable 20 können z. B. Kontaktfedern realisiert sein, welche mit jedem Wechsel des Disposable 20 ausgetauscht werden.

Sobald ein Disposable 20 an ein Reusable 10 gekoppelt wird, startet der Mikroprozessor 13 im Reusable 10 aufgrund der angelegten Disposable-Batteriespannung am in Figur 4 gezeigten Anschluss 1. Das Aufwecken des Reusables 10 ist beispielsweise auch mit einer teilentladenen Batterie 21 möglich. Wird ein Disposable 20 mit komplett entladener Batterie 21 an einem Reusable 10 angeschlossen, so ist kein Aufwecken möglich.

Der Akku 11 des Reusable 10 wird über die Batterie 21 des Disposable 20 geladen oder nachgeladen. Die Ladung des Akkus 11 erfolgt im Normalfall mit möglichst konstantem Ladestrom, wodurch die aus der Batterie 21 entnehmbare Kapazität maximiert wird. Der Ladestrom wird z. B. an den Ladezustand des Akkus 11 angepasst. Über die angelegte Batteriespannung kann auch die Anwesenheit des Disposables 20 am Reusable 10 überprüft werden.

Im Disposable 20 ist ein Mikrocontroller MCU enthalten, welcher zum Lesen und Schreiben von das Disposable 20 betreffenden Informationen ausgelegt ist, wie z. B. Version, Status des Insertionsmechanismus 24, Reservoirgröße oder Ablaufdatum der im Reservoir 22 enthaltenen Substanz. Der Mikrocontroller MCU kann über den Anschluss 2 oder 4 mit dem über die Schnittstelle gekoppelten Reusable 10 kommunizieren.

Optional kann ein Disposable 20 zu einem beliebigen Zeitpunkt entwertet werden, wenn z. B. schon eine oder mehrere Füllstandserkennungen durchgeführt wurden oder beispielsweise erst nach dem Auslösen des Insertionsmechanismus 24. Wird von einem Reusable 10 ein entwerteter Disposable 20 erkannt, so wird er zurückgewiesen, d. h. das Reusable 10 stellt für das als entwertet erkannte Disposable 20 keine Abgabefunktionen, wie z. B. Betrieb des Motors 12, zur Verfügung.

Das Signal zum Auslösen des Kanülen- oder Nadelinsertionsmachanismus 24 kann über eine Kommunikationsschnittstelle z. B. bei Anschluss 4 an die MCU übertragen werden, welche den Nadeleinstichmechanismus 24 steuern kann.

Die Batterie 21 im Disposable kann, muss aber nicht, dafür ausgelegt sein die Energie zur Auslösung des Nadelinsertionsmechanismus 24 zu liefern. Diese Energie kann z. B. über den Anschluss 2 vom Akku 11 des Reusable 10 geliefert werden.

Im Lagerzustand ist der Schalter S zur Speisung des Nadelinsertionsmechanismus 24 geschlossen. Während dem Einstechen der Nadel oder Kanüle 23 wird der Schalter S, der mit der Veränderung der Nadelposition N schalten kann und z. B. mit der Kanüle 23 in Verbindung steht oder mechanisch gekoppelt ist, geöffnet. Durch die Spannungsmessung zwischen dem Nadeleinstechmechanismus 24 und dem Schalter S kann einerseits die Spannung am Anschluss 2 vor dem Auslösen der Nadel oder Kanüle 23 geprüft werden und andererseits der Status des Schalters S erkannt werden. Bei geschlossenem Schalter S tritt ein Spannungseinbruch beim Einschalten des Nadel- oder Kanüleneinstechmechanismus 24 auf. Im offenen Zustand ist keine Spannung messbar. Die MCU speichert den Status und das Reusable 10 kann jederzeit den Status über den Anschluss 4 auslesen.

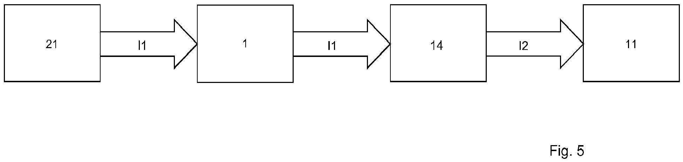

Figur 5 zeigt schematisch das Energieversorgungskonzept der Pumpe, welche zeigt, dass der Betrieb der Elektronik, wie z. B. der Steuerung 13 des Reusable 10, durch den Akku 11 im Reusable 10 sichergestellt werden kann. Die Batterie 21 im Disposable 20 wird zum Aufladen oder Nachladen des Akkus 11 des Reusable 10 mit dem Ladestrom I1 verwendet. Dabei kann mit einer Ladeelektronik 14 z. B. eine Spannungsanpassung oder Ladeautomatik realisiert werden. Beispielsweise kann je nach Betriebsart der Ladeelektronik 14 der von der Batterie 21 gelieferte Ladestrom I1 größer, gleich oder geringer als der zur Aufladung des Akkus 11 bereitgestellte Ladestrom I2 sein. Somit können alle Verbraucher mit hohem Energiebedarf, wie z. B. der Motor 12 oder Antrieb zur Insulinförderung, ein optional vorgesehener Summer oder Signalisierungsvorrichtung, die Auslösung des Kanüleninsertionsmechanismus 24, vom Akku 11 des Reusable 10 mit elektrischer Energie versorgt werden. Die Batterie 21 des Disposable 20 kann kontinuierlich und verlustarm zum Aufladen des Akkus 11 entladen werden.

Falls der Akku 11 z. B. für eine vorgesehene Therapie ungenügend geladen ist, wird der Akku 11 zunächst über eine schnelle Ladung nachgeladen. Falls die Batterie 21 beim Erreichen des minimalen Ladezustandes des Akkus 11 für die Förderung der gewünschten Reservoirmenge des angeschlossenen Disposables 20 nicht hinreichend geladen ist, kann ein Austausch des Disposables 20 erforderlich sein.

Vorzugsweise ist die Kapazität der Batterie 21 größer als die, welche für eine vorgegebene Therapiedauer von beispielsweise 3 Tagen und eine maximale Füllmenge des Reservoirs 22 benötigt wird. Hierdurch kann der Ladestand des Akkus 11 mit jedem Disposable 20 bzw. jeder neuen Batterie 21 erhöht werden.

Die Batterie 21 im Disposable 20 kann z. B. bei einem Ausfall des Akkus 11 im Reusable 10 als Energiequelle zur Alarmierung des Benutzers dienen und z. B. die Energie zur Alarmierung über ein Smartphone 30 zur Verfügung stellen.

### Funktionalität SmartSwap

In Verbindung mit der oben beschriebenen Pumpe oder allgemein bei Verwendung von mindestens zwei Pumpen kann eine Austausch- oder Fortsetzungs-Funktion in der Steuerung und Kommunikation der Pumpen integriert sein, um einen Benutzer beim nahtlosen Wechsel zwischen zwei Pumpen zu unterstützen.

Dabei kann, während eine Pumpe noch aktiv ist, eine zweite Pumpe z. B. befüllt, aktiviert und am Körper angebracht werden. Ist die erste Pumpe leer, wird dies von der ersten Pumpe festgestellt und alle relevanten Betriebsparameter, wie z. B. laufende Basalraten, Boli sowie optional weitere Einstellungen von der zuletzt noch aktiven ersten Pumpe auf die neue zweite Pumpe übertragen und dieser das Signal gegeben, basierend auf den übertragenen Daten eine Substanzabgabe wenn möglich nahtlos oder unterbrechungsfrei fortzusetzen.

Somit kann z. B. eine in einer Pumpe enthaltene Substanz praktisch restlos aufgebraucht werden und ein diskreter Wechsel von einer Pumpe zur anderen ermöglicht werden, ohne dass ein Benutzer eingreifen muss, um den Funktionsübergang von einer Pumpe zur anderen zu initiieren.

## Patentansprüche

1. Einwegmodul (Disposable) (20), welches mit einem Mehrwegmodul (Reusable) (10) gekoppelt werden kann, um im gekoppelten Zustand eine Pumpe zur dosierten Abgabe einer Substanz an einen Anwender zu bilden, mit einem Gehäuse, das folgende Komponenten umgibt:
- ein Reservoir (22) zur Aufnahme der abzugebenden Substanz; und
- eine ausfahrbare Kanüle (23), insbesondere eine Hartkanüle und/oder eine Softkanüle, die zumindest nach der Insertion oder dauerhaft, also auch vor einem Insertionsvorgang, mit dem Reservoir (22) in Fluidverbindung steht oder stehen.

2. Einwegmodul (20) nach Anspruch 1, wobei innerhalb des Einwegmodul-Gehäuses ein Kanülen-Insertionsmechanismus (24) vorgesehen ist, welcher einen Kraft- oder Energiespeicher, z.B. eine Feder, enthält, welcher die Kanüle (23) zur Insertion antreiben oder aus dem Gehäuse herausbewegen kann.

3. Einwegmodul (20) nach einem der vorhergehenden Ansprüche, wobei der Kanülen-Insertionsmechanismus (24) die Kanüle (23) automatisch oder selbstständig aus dem Einwegmodul (20) ausstoßen und/oder automatisch oder selbstständig einen Insertionsvorgang der Kanüle (23) in einen Anwender durchführen kann.

4. Einwegmodul (20) nach einem der vorhergehenden Ansprüche mit einer Batterie (21), welche elektrische Energie für das Mehrwegmodul (10) zur Verfügung stellt, die z.B. benötigt wird, um die Substanz aus dem Reservoir (22) abzugeben.

5. Einwegmodul (20) nach dem vorhergehenden Anspruch, wobei die Batterie (21) fest oder unlösbar mit dem Einwegmodul (20) verbunden ist.

6. Einwegmodul nach einem der beiden vorhergehenden Ansprüche, wobei die Batterie (21) aus einer oder mehr Einwegbatterien oder Knopfzellen besteht.

7. Mehrwegmodul (Reusable) (10) mit einem aufladbaren Energiespeicherelement oder Akku (11) und einem Antrieb (12) und einer Steuerung (13), die vom Akku (11) mit elektrischer Energie versorgt werden können und mit einer mechanischen oder elektrischen Schnittstelle zur Verbindung mit dem Einwegmodul (20) nach einem der vorhergehenden Ansprüche 3 bis 6, um den Kanülen-Insertionsmechanismus (24) zur Insertion der Kanüle (22) auszulösen.

8. Mehrwegmodul nach dem vorhergehenden Anspruch, wobei der Akku (11) fest oder auswechselbar mit dem Mehrwegmodul (10) verbunden ist.

9. Mehrwegmodul nach einem der beiden vorhergehenden Ansprüche, wobei die Steuerung (13) mit einem externen Steuergerät (30) kabellos oder über Funk zum Austausch von Steuersignalen kommunizieren kann.

10. Pumpe mit einem Einwegmodul (20) nach einem der Ansprüche 1 bis 6 und einem Mehrwegmodul (10) nach einem der Ansprüche 7 bis 9.

11. Pumpe nach dem vorhergehenden Anspruch, wobei die Pumpe eine Patch-Pumpe ist.

12. Schnittstelle zwischen einem Einwegmodul (Disposable) (20) nach einem der Ansprüche 1 bis 6 und einem Mehrwegmodul (Reusable) (10) nach einem der Ansprüche 7 bis 9, welche so ausgestaltet ist, dass eine elektrische Energie oder eine Ladestrom vom Disposable (20) zum Aufladen eines elektrischen Energiespeichers oder Akkus (11) im Reusable (10) übertragen werden kann und wobei die vom Reusable (10) benötigte elektrische Energie sowohl vom Disposable (20) als auch vom Akku (11) bezogen werden kann, sodass im Normalbetrieb bei nicht defektem Akku (11) die vom Reusable (10) benötigte elektrische Energie aus dem Akku (11) bezogen werden kann und bei gestörter oder unterbrochener Stromversorgung durch den Akku (11) die vom Reusable (10) benötigte elektrische Energie vom Disposable (20) oder dessen Einwegbatterie oder Einwegbatterien (21) bezogen werden kann, um eine Redundanz der Stromversorgung sicherzustellen.

13. Verfahren zum Betreiben einer Pumpe nach Anspruch 10 oder 11, wobei eine Batterie (21) des Einwegmoduls (20) mit einem Akku (11) des Mehrwegmoduls (10) gekoppelt wird und diesen auflädt und wobei die Energieversorgung bei funktionsfähigem Akku (11) von diesem zur Verfügung gestellt wird und bei nicht funktionsfähigem Akku (11) vom Disposable (20) oder dessen Batterie (21) zur Verfügung gestellt wird.

14. Verfahren nach dem vorhergehenden Anspruch, wobei die in der Batterie (21) des Einwegmoduls (20) enthaltene Energiemenge größer ist als die Energiemenge, die zur Abgabe der gesamten im Reservoir (22) des Einwegmoduls (20) enthaltenen Substanz erforderlich ist.
